# EUROPEAN PATENT APPLICATION

(11) **EP 1 726 287 A1**
(43) Date of publication of application: **29.11.2006**
(21) Application number: 05719374.0
(22) Date of filing: 22.02.2005
(51) Int. Cl.: A61K 8/18

(54) **STICK COSMETIC AND PROCESS FOR PRODUCING THE SAME**

(30) Priority: 16.03.2004 JP 2004074665
(71) Applicant: MITSUBISHI PENCIL Co., Ltd., Tokyo 140-8537 (JP)
(72) Inventor: BANZAI, Satoru, Mitsubishi Pencil Co., Ltd., Fujioka-shi, Gunma 375-8501 (JP)
(74) Representative: Luderschmidt, Schüler & Partner
(86) International application number: PCT/JP2005/002784
(87) International publication number: WO 2005/087180

(57) **Abstract**

In order to provide a stick type cosmetic in which applying feeling can freely be set either hard or soft according to a size and a distribution of mesopores or the kind of a material while maintaining a satisfactory color developing property and satisfactory drawn line intensity and which is excellent in mechanical strength (flexural strength, tensile strength and impact strength) and can actualize multicolor, the stick type cosmetic is **characterized by** comprising a mesoporous material as a skeleton, and a production process for the same is **characterized by** producing the stick type cosmetic by synthesizing the mesoporous material at low temperature by means of microwave heating and ultrasonic cleaning.

## Description

### Technical Field

The present invention relates to a stick type cosmetic for a stick container used primarily for cosmetics, a stick type cosmetic for a wood case and a paper-covered stick type cosmetic, more specifically to a stick type cosmetic in which a mesoporous material is contained as a skeleton so that applying feeling can freely be set either hard or soft according to a size and a distribution of the mesopores or the kind of the material while maintaining a satisfactory color developing property and satisfactory drawn line intensity and which is excellent in mechanical strength (flexural strength, tensile strength and impact strength) and can actualize multicolor and to a production process for the same.

### Background Art

In general, important characteristics required in a stick type cosmetic are good applying feeling, actualization of multicolor, a good color developing property of the drawn lines and large mechanical strength.

Known as a stick type cosmetic satisfying the above characteristics are, for example, stick type cosmetics blended with a silicone oil, an ester oil comprising fatty acid and aliphatic alcohol and having an intramolecular branch structure and a solidifying point of 0°C or less, a methylhydrogenpolysiloxane-treated pigment and solid oils & fats as essential components (refer to, for example, patent document 1).

In the above stick type cosmetics, oil components such as the oils & fats and the silicone oil are contained, and these materials exert a strong effect on the strength, so that involved therein is the problem that when powdery (applying feeling obtained when powder is solidified), soft and good applying feeling is set, the strength is reduced.

On the other hand, stick type cosmetics which aim at powdery applying feeling without using waxes are researched. Known are, for example, those using water-soluble adhesive pastes such as carboxymethyl cellulose (CMC) (refer to, for example, patent document 2) and those using gypsum (refer to, for example, patent document 3).

However, in the stick type cosmetics using the above binders, the binders are extremely hardened, and therefore the applying feeling to the skin is very hard. Also, slender ones such as eyebrows and eyeliners are very fragile, and those in which an amount of the binders is increased in order to obtain satisfactory mechanical strength can not be applied at all. That is, in the stick type cosmetics using the water-soluble adhesive pastes such as CMC and gypsum for the binders in place of waxes in order to obtain powdery use feeling, it is difficult to satisfy satisfactory mechanical strength and good applying feeling at the same time.

Further, stick type cosmetics which satisfy satisfactory mechanical strength and good applying feeling at the same time without using waxes are researched as well, and known are those in which clay is used for a binder and sintered (refer to, for example, patent document 4) and those in which a pore-forming material is used to prepare a more porous sintered body (refer to, for example, patent document 5).

The invention disclosed in the patent document 4 described above is a stick type cosmetic comprising an inorganic pigment dispersed in a powder form in a sintered body of clay which is subjected to sintering treatment to thereby form a porous skeleton. In respect to a porous body, described in the above document is "the larger the porosity is, the more the touch and the applying property tend to be improved, and the smaller the porosity is and the more the minuteness is enhanced, the more the strength tends to be increased", and what has been disclosed in the above document ends up with a sintered body having a porosity of 50 to 90 %. That is, it is described that while a satisfactory mechanical strength is obtained in the sintered body of clay, it is hard and difficult to be applied on the skin, but a stick type cosmetic which satisfies satisfactory mechanical strength and good applying feeling at the same time is obtained by preparing a porous sintered body.

However, it is indispensable for satisfying good applying feeling and satisfactory mechanical strength at the same time to control a porosity. The porosity is controlled, as described in the foregoing patent document 4, only by changing a temperature in sintering treatment and a use proportion of the powder pigment, and it is very difficult to obtain the preferred sintered body having a porosity of 50 to 90 % which is described in the above patent document 4.

Further, in those disclosed in the patent document 5 described above in which a pore-forming material is used in sintering to prepare a more porous sintered body, a stick type cosmetic which satisfies satisfactory mechanical strength and good applying feeling at the same time is not obtained.

Further, known as such a stick type cosmetic is a stick type cosmetic (refer to, for example, a patent document 6) prepared by sintering essential components comprising 1 to 10 % by weight of at least one selected from the group consisting of bentonite, smectite, montmorillonite, beidelite, nontronite, hectorite and saponite, an inorganic extender pigment and an inorganic color pigment in a temperature range of 300 to 1000° C to obtain an inorganic powder in which carbides do not contain at all, and molding a composition comprising the inorganic powder and water, drying the molded composition and then subjecting it to heat treatment at 300 to 1000°C. Clay is sintered to become a binder as is the case with what has been described above, and therefore when it is blended with such a material that soft applying feeling is obtained, the problem that the strength is reduced is involved therein.

When clay is sintered to become a binder as described above, an organic pigment and a dye are discolored, and therefore only pigments having high heat resistance such as inorganic pigments can be used, so that involved therein is the problem that it is difficult to actualize sufficient multicolor and obtain a satisfactory color developing property.

On the other hand, known as mesoporous powder using for cosmetics are block mesoporous powder (referto, for example, patent document 7) in which silicon oxide is provided on an outer shell of an aperture and in which a depth of the aperture is 50 to 300 nm and bar-like mesoporous powder (refer to, for example, patent document 8) which comprises silicon oxide as a principal component and has an outer diameter of 20 to 200 nm and in which mesopores are extended in a longitudinal direction thereof.

However, in the mesoporous powder described in the above patent documents, stick type cosmetics are not objectives, and described therein are processes in which such powder is prepared and in which the powder is solidified or mixed with fluid to prepare rouges and foundations. Accordingly, the problem that it is difficult to mold powders into a stick form and maintain the form is involved therein.
Patent document 1: Japanese Patent Publication No. 169714/1987 (claims, examples and others)
Patent document 2: Japanese Patent Application Laid-Open No. 44305/1984 (claims, examples and others)
Patent document 3: Japanese Patent Application Laid-Open No. 93014/1984 (claims, examples and others)
Patent document 4: Japanese Patent Application Laid-Open No. 176513/1986 (claims, examples and others)
Patent document 5: Japanese Patent Application Laid-Open No. 197507/1986 (claims, examples and others)
Patent document 6: Japanese Patent Application Laid-Open No. 188518/1996 (claims, examples and others)
Patent document 7: Japanese Patent Application Laid-Open No. 152317/1998 (claims, examples and others)
Patent document 8: Japanese Patent Application Laid-Open No. 100208/1999 (claims, examples and others)

### Disclosure of the Invention

### Problems to be solved by the Invention

In light of the problems on the conventional arts described above, the present invention intends to solve them, and an object thereof is to provide a stick type cosmetic which maintains mechanical strength while providing soft and good applying feeling and which provides a vivid color developing property and color intensity and can actualize multicolor and a production process for the same.

### Means for solving the problems

Intensive researches repeated by the present inventors in order to solve the problems of the conventional arts described above have resulted in finding that a stick type cosmetic meeting the object described above and a production process for the same can be obtained by at least adding a mesoporous material as a skeleton on a surface or an interface of powder such as an extender material constituting a lead, and thus the present invention has come to be completed.

That is, the present invention comprises in the following items (1) to (6).
(1) A stick type cosmetic comprising a mesoporous material as a skeleton.
(2) The stick type cosmetic as described in the above item (1), wherein the mesoporous material is at least one selected from the group consisting of oxide ceramics, nitride ceramics, phosphate ceramics, carbide ceramics, silicate ceramics and boride ceramics.
(3) The stick type cosmetic as described in the above item (1), wherein the mesoporous material is a composite material of at least one selected from the group consisting of oxide ceramics, nitride ceramics, phosphate ceramics, carbide ceramics, silicate ceramics and boride ceramics and an organic substance and/or a metal.
(4) The stick type cosmetic as described in any one of the above items (1) to (3), wherein a mesopore of the mesoporous material has a diameter falling in a range of 2 nm to 1000 nm.
(5) The stick type cosmetic as described in any one of the above items (1) to (4), wherein it is s cosmetic pencil.
(6) A production process for a stick type cosmetic, wherein the stick type cosmetic as described in any one of the above items (1) to (5) is produced by synthesizing a mesoporous material at low temperature by means of microwave heating or microwave heating and ultrasonic cleaning.

### Effect of the invention

According to the present invention, a mesoporous material which is a binder is contained as a skeleton after molding a material, and therefore provided are a stick type cosmetic in which applying feeling can freely be set either hard or soft according to a size and a distribution of mesopores or the kind of the material while maintaining a satisfactory color developing property and satisfactory drawn line intensity and which is excellent in mechanical strength (flexural strength, tensile strength and impact strength) and can actualize multicolor and a production process for the same.

Also, a stick type cosmetic providing further powdery and good applying feeling is prepared by using at least one selected from the group consisting of oxide ceramics, nitride ceramics, phosphate ceramics, carbide ceramics, silicate ceramics and boride ceramics as the mesoporous material to constitute a skeleton.

Further, in the process of the present invention, the stick type cosmetic can be produced by synthesizing the mesoporous material at low temperature by means of microwave heating or microwave heating and ultrasonic cleaning, and therefore provided is a production process for a stick type cosmetic in which various pigments can be used without bringing about degradation by heat treatment and in which color development is less damaged.

### Best Mode for Carrying Out the Invention

The embodiment of the present invention shall be explained below in details.

The stick type cosmetic of the present invention is characterized by comprising a mesoporous material as a skeleton. Also, the production process for a stick type cosmetic according to the present invention is characterized in that the stick type cosmetic is produced by synthesizing a mesoporous material at low temperature by means of microwave heating or microwave heating and ultrasonic cleaning.

The stick type cosmetic of the present invention is, as described above, characterized by comprising a mesoporous material as a skeleton and assumes a constitution in which raw materials such as a mesoporous material (rawmaterial), an extender material, a colorant such as a pigment and a surfactant are molded and in which the mesoporous material is then contained as a skeleton (binder).

The mesoporous material (raw material) used in the present invention shall not specifically be restricted as long as it is usually classified into a mesoporous material, and any materials can be used.

For example, a mesoporous material (raw material) comprising silicon oxide as a principal material can be used. Four kinds of the following synthetic processes 1) to 4) according to the combinations of a silicon oxide source and a template are known as a synthetic process for the above mesoporous material.
1) A process described in U.S. Patent 3556725, Published Japanese Translation of PCT international publication No. 503499/1993 and Japanese Patent Application Laid-Open No. 34607/1996, and a mesoporous material is synthesized in an alkaline region, wherein amorphous silicon oxide powder, an alkyl silicate aqueous solution or activated silicon oxide is used as a silicon oxide source, and an ammonium salt having a long alkyl group or a phosphonium salt is used as a template.
2) A process described in Japanese Patent Application Laid-Open No. 238810/1992, and a mesoporous material is synthesized by an ion exchange method, wherein stratiform silicate such as kanemite is used as a silicon oxide source, and a long chain alkylammonium cation is used as a template.
3) A process described in U.S. Patent 5672556, wherein alkoxide such as tetraethoxysilane is used as a silicon oxide source, and a surfactant such as alkylamine is used as a template.
4) A process reported in H. Minakuchi, K. Nakanishi et al, Anal. Chem. p. 3489 to 3501, 69 (1996) and J. Porous, Mater. 467-112 (1997), wherein alkoxysilane such as tetraethoxysilane and tetramethoxysilane is used as a silicon oxide source, and it is phase-separated from a water-soluble polymer and a surfactant used as a template to obtain mesoporous silica.

The above synthetic processes can suitably be selected according to the embodiments of the stick type cosmetic of the present invention and used.

Various ceramic materials can be used for the mesoporous material (raw material) used in the present invention, and capable of being used is, for example, any of oxide ceramics of metals such as silicon, titanium, zirconium and aluminum, nitride ceramics, phosphate ceramics, carbide ceramics, silicate ceramics and boride ceramics. They are used alone or can be used in a mixture of two or more kinds thereof, and they are suitably selected according to the shape of the aimed molded article and a molding method.

Further, materials (composite material) obtained by compounding at least one selected from the group consisting of the oxide ceramics, the nitride ceramics, the phosphate ceramics, the carbide ceramics, the silicate ceramics and the boride ceramics each described above with an organic substance and/or a metal can be used as well from the viewpoint of further providing the ceramic materials described above with preferred characteristics such as flexibility and a water repellent property.

The composite materials compounded with an organic substance includes, for example, organic-inorganic hybrid materials obtained by compounding a methyl group or a phenyl group into inorganic polymers and organic-inorganic hybrid materials obtained by mixing organic polymers such as polyvinyl chloride, polyethylene glycol, polyvinylpyrrolidone and methyl cellulose, and they can suitably be selected considering the physical characteristics and the molding conditions but shall not be restricted thereto.

The composite materials compounded with a metal includes, for example, those on which a rust preventive coating is formed by adding at least one metal ion selected from metal cations of Ti, Zr, Ce, Sr, V, W and Mo and oxy metal anions and fluorometal anions thereof in synthesizing the mesoporous material and those provided with a catalyst function by adding a catalyst metal such as Pt, and they can suitably be selected considering the characteristics which are desired to be added and the physical conditions but shall not be restricted thereto.

A content of the above mesoporous materials (raw materials) is preferably 0.1 to 50 % by weight, more preferably 0.1 to 20 % by weight based on the total amount of the stick type cosmetic blend composition.

If a content of the above mesoporous materials (rawmaterials) is less than 0.1 by weight, the molding property and the homogeneity in molding are notably damaged, and it is difficult to obtain a molded article having a certain shape. Further, strength of the molded article is markedly weakened. On the other hand, if a content of the mesoporous materials (raw materials) exceeds 50 % by weight, the strength is enhanced, but brought about is the negative effect that the cosmetic is hard and can not be applied or is less liable to be abraded.

The extender material used for the stick type cosmetic of the present invention shall not specifically be restricted as long as it is used for conventional stick type cosmetics, and any materials can be used. Capable of being used are, for example, white extender materials such as boron nitride, kaoline, talc, mica and calcium carbonate, and colored extender materials can be used as well depending on a hue of the stick type cosmetic. It is a matter of course that mixtures of several kinds thereof can be used as well. Particularly preferably, it includes boron nitride, kaoline and talc from the viewpoint of the physical properties and the form. Further, the mesoporous materials described above can be the extender material.

Any compounds can be used as the surfactant used in the present invention as long as they are usually classified into surfactants, and either ionic surfactants or non-ionic surfactants can be used.

The ionic surfactants include, for example, anionic surfactants such as carboxylic acid salts, sulfuric acid ester salts, sulfonic acid salts and phosphoric acid ester salts, cationic surfactants such as alkylamine salts, quaternary ammonium salts and alkylpyridinium salts and amphoteric surfactants such as amino acid types, betaine types, sulfonic acid salt types and phosphoric acid ester types. The non-ionic surfactants include, for example, polyethylene glycol types and polyhydric alcohol types. However, they shall not be restricted thereto, and the above surfactants can be used alone or in a mixture of two or more kinds thereof.

The colorant used in the present invention includes, for example, pigments such as titanium oxide, black iron oxide, carbon black, Prussian blue, ultramarine blue, blue No. 1, red iron oxide, yellow iron oxide, chromium oxide, chromium hydroxide, zinc oxide, zirconium oxide, cobalt oxide, fish scale flake, bismuth oxychloride, titanium dioxide coated mica, blue No. 2, blue No. 404, red No. 2, red No. 3, red No. 102, red No. 104, red No. 105, red No. 106, yellow No. 4, yellow No. 5 and green No. 3. They can be used alone or in a mixture of two or more kinds thereof.

The stick type cosmetic of the present invention containing, as a skeleton, the mesoporous material which is the binder is produced by a low temperature process, wherein kneaded are the mesoporous material (raw material), the extender material, the colorant such as the pigment and the surfactant each described above, and the kneaded composition is molded into a prescribed shape and then sintered and eluted with a solvent and heated by means of microwave.

To be specific, kneaded are the raw materials (blend composition) such as the mesoporous material (raw material) [including a solution for forming the mesoporous material], the extender material, the colorant such as the pigment and the surfactant; the kneaded composition is extrusion-molded in a fine line by an extruding machine and dried; it is then heated by means of microwave or subjected to ultrasonic cleaning with water (distilled water, refined water) and then heated by means of microwave, whereby the aimed stick type cosmetic in which a template is removed and in which the mesoporous material working as the binder is contained as a skeleton can be produced. The conditions (frequency, irradiation time and others) of microwave heating are varied depending on the uses, the shape and the kind of raw material of the stick type cosmetic, and in the case of the stick type cosmetic having a diameter of about 2 mm, the sample of about 5 g is heated at 0.30 to 30 GHz and 700 W for 0.5 to 3 minutes.

In the present invention, the mesoporous material can be produced, as described above, by synthesizing at low temperature (100°C or lower) by microwave heating or microwave heating and ultrasonic cleaning, and therefore various pigments including pigments having low heat resistance can be used without bringing about deterioration caused by heat treatment, whereby produced is the stick type cosmetic in which color development is less liable to be damaged and which actualizes satisfactory multicolor and has a vivid color developing property.

The mesopores of the mesoporous material in the stick type cosmetic obtained preferably have a diameter falling in a range of 2 nm to 1000 nm, more preferably 50 nm to 500 nm from the viewpoint of obtaining the stick type cosmetic in which an applying feeling is soft and good and which has a vivid color developing property and drawn line intensity while maintaining mechanical strength. The mesopores described above can be measured (including examples described later) by means of a pore size distribution measuring apparatus by mercury porosimetry (manufactured by Yuasa Ionics Inc.).

In the present invention thus constituted, the mesoporous material working as the binder is contained as a skeleton after molding the materials, and therefore obtained are the stick type cosmetic in which applying feeling can freely be set either hard or soft according to a size and a distribution of mesopores or the kind of the material while maintaining a satisfactory color developing property and satisfactory drawn line intensity and which is excellent in mechanical strength (flexural strength, tensile strength and impact strength) and can actualize multicolor and the production process for the same.

The stick type cosmetic thus obtained can suitably be applied to a stick type cosmetic for a stick container, a stick type cosmetic for a wood case and a paper-covered stick type cosmetic, and in particular, it can suitably be applied as a cosmetic pencil.

### Examples

Next, the present invention shall be further explained with reference to examples and comparative examples, but the present invention shall not be restricted to the following examples.

### Example 1

Mixed were 300 g of ion-exchanged water, 300 g of cetyltrimethylammonium bromide as a surfactant and 25 g of HCl of 0.1 mol/l, and the solution was stirred at 30°C for 30 minutes (solution 1). Then, a mixed solution of 50 g of tetraethyl orthosilicate (TEOS) as an Si source and 50 g of titanium isopropoxide as a Ti source was added to the solution 1 described above, and the solution was stirred at 30°C for 2 hours to prepare a solution A for forming a mesoporous material. The solution A was used to obtain the following blend composition and a stick type cosmetic according to the following production process.

### Blend composition:

| | |
|---|---|
| Solution A | 30.0 % by weight |
| Prussian blue | 24.0 % by weight |
| Titanium oxide | 6.0 % by weight |
| Kaoline | 20.0 % by weight |
| Boron nitride | 20.0 % by weight |

The blend composition was kneaded, and the kneaded composition was extrusion-molded in a fine line by means of an extruding machine. After dried in the air at 50 °C for 24 hours, the molded article was subjected to ultrasonic cleaning with distilled water for one hour and then heated by microwave (2.45 GHz, 700 W) for 3 minutes to obtain a light blue stick type cosmetic having a diameter of 2.0 mm.

### Example 2

No. 3 sodium silicate (SiO₂ = 29.1 %, Na₂O = 9.45 %) of 20 g was diluted with 100 g of ion-exchanged water, and the solution was allowed to pass through a column filled with a cation exchange resin (Amberlite IR-120B) which was converted in advance into a II+ type to collect 100 g of activated silica. On the other hand, an aqueous solution in which 5.6 g of octadecyltrimethylammonium chloride was dissolved was prepared, and the whole amount of the activated silica sol described above was added thereto little by little in about 5 hours, whereby a solution B for forming a mesoporous material was prepared. The solution B was used to obtain the following blend composition and a stick type cosmetic according to the following production process.

### Blend composition:

| | |
|---|---|
| Solution B | 30.0 % by weight |
| Prussian blue | 14.0 % by weight |
| Titanium oxide | 6.0 % by weight |
| Kaoline | 30.0 % by weight |
| Boron nitride | 20.0 % by weight |

The blend composition was kneaded, and the kneaded composition was extrusion-molded in a fine line by means of an extruding machine. After dried in the air at 50°C for 24 hours, the molded article was heated up to 100°C and dried for 5 hours, and it was subjected to ultrasonic cleaning with distilled water for one hour and then heated by microwave (2.45 GHz, 700 W) for 3 minutes to obtain a light blue stick type cosmetic having a diameter of 2.0 mm.

### Example 3

Tetraethoxysilane of 50 g was diluted with 50 g of ethanol, and 50 g of polyethylene glycol (average molecular weight: 400) was slowly dropwise added thereto. After mixed for one hour, 100 g of a 0.1N-HCl aqueous solution was dropwise added thereto and mixed for 6 hours to prepare a solution C for forming a mesoporous material. The solution C was used to obtain the following blend composition and a stick type cosmetic according to the following production process.

### Blend composition:

| | |
|---|---|
| Solution C | 30.0 % by weight |
| Prussian blue | 20.0 % by weight |
| Titanium oxide | 5.0 % by weight |
| Kaoline | 15.0 % by weight |
| Boron nitride | 30.0 % by weight |

The blend composition was kneaded, and the kneaded composition was extrusion-molded in a fine line by means of an extruding machine. After dried in the air at 80°C for 24 hours, the molded article was further subjected to ultrasonic cleaning with distilled water for one hour and then heated by microwave (2.45 GHz, 700 W) for 3 minutes to obtain a light blue stick type cosmetic having a diameter of 2.0 mm.

### Example 4

Tetraethoxysilane of 50 g was diluted with 50 g of ethanol, and 100 g of polyethylene glycol (average molecular weight: 400) was slowly dropwise added thereto. After mixed for one hour, 100 g of a 0.1N-HCl aqueous solution was dropwise added thereto and mixed for 6 hours to prepare a solution D for forming a mesoporous material. The solution D was used to obtain the following blend composition and a stick type cosmetic according to the following production process.

### Blend composition:

| | |
|---|---|
| Solution D | 30.0 % by weight |
| Prussian blue | 20.0 % by weight |
| Titanium oxide | 5.0 % by weight |
| Kaoline | 15.0 % by weight |
| Boron nitride | 30.0 % by weight |

The blend composition was kneaded, and the kneaded composition was extrusion-molded in a fine line by means of an extruding machine. After dried in the air at 80 °C for 24 hours, the molded article was further subjected to ultrasonic cleaning with distilled water for one hour and then heated by microwave (2.45 GHz, 700 W) for 3 minutes to obtain a light blue stick type cosmetic having a diameter of 2.0 mm.

### Example 5

Tetraethoxysilane of 50 g was diluted with 50 g of ethanol, and 100 g of polyethylene glycol (average molecular weight: 600) was slowly dropwise added thereto. After mixed for one hour, 100 g of a 0.1N-HCl aqueous solution was dropwise added thereto and mixed for 6 hours to prepare a solution E for forming a mesoporous material. The solution E was used to obtain the following blend composition and a stick type cosmetic according to the following production process.

### Blend composition:

| | |
|---|---|
| Solution E | 30.0 % by weight |
| Prussian blue | 20.0 % by weight |
| Titanium oxide | 5.0 % by weight |
| Kaoline | 15.0 % by weight |
| Boron nitride | 30.0 % by weight |

The blend composition was kneaded, and the kneaded composition was extrusion-molded in a fine line by means of an extruding machine. After dried in the air at 80°C for 24 hours, the molded article was further subjected to ultrasonic cleaning with distilled water for one hour and then heated by microwave (2.45 GHz, 700 W) for 3 minutes to obtain a light blue stick type cosmetic having a diameter of 2.0 mm.

### Example 6

Tetraethoxysilane of 50 g was diluted with 50 g of ethanol, and 100 g of polyethylene glycol (average molecular weight: 1000) was slowly dropwise added thereto. After mixed for one hour, 100 g of a 0.1N-HCl aqueous solution was dropwise added thereto and mixed for 6 hours to prepare a solution F for forming a mesoporous material. The solution F was used to obtain the following blend composition and a stick type cosmetic according to the following production process.

### Blend composition:

| | |
|---|---|
| Solution F | 30.0 % by weight |
| Prussian blue | 20.0 % by weight |
| Titanium oxide | 5.0 % by weight |
| Kaoline | 15.0 % by weight |
| Boron nitride | 30.0 % by weight |

The blend composition was kneaded, and the kneaded composition was extrusion-molded in a fine line by means of an extruding machine. After dried in the air at 80°C for 24 hours, the molded article was further subjected to ultrasonic cleaning with distilled water for one hour and then heated by microwave (2.45 GHz, 700 W) for 3 minutes to obtain a light blue stick type cosmetic having a diameter of 2.0 mm.

### Example 7

Tetraethoxysilane of 50 g was diluted with 50 g of ethanol, and 100 g of polyethylene glycol lauryl ether was slowly dropwise added thereto. After mixed for one hour, 100 g of a 0 .1N-HCl aqueous solution was dropwise added thereto and mixed for 6 hours to prepare a solution G for forming a mesoporous material. The solution G was used to obtain the following blend composition and a stick type cosmetic according to the following production process.

### Blend composition:

| | |
|---|---|
| Solution G | 30.0 % by weight |
| Prussian blue | 20.0 % by weight |
| Titanium oxide | 5.0 % by weight |
| Kaoline | 30.0 % by weight |
| Boron nitride | 15.0 % by weight |

The blend composition was kneaded and further dispersed by means of three rolls, and then the kneaded composition was extrusion-molded in a fine line by means of an extruding machine. After dried in the air at 80°C for 24 hours, the molded article was subjected to ultrasonic cleaning with distilled water for one hour and then heated by microwave (2.45 GHz, 700 W) for 3 minutes to obtain a light blue stick type cosmetic having a diameter of 2.0 mm.

### Example 8

### Blend composition:

| | |
|---|---|
| Solution G (Example 7) | 30.0 % by weight |
| Blue No. 1 | 5.0 % by weight |
| Ultramarine blue | 10.5 % by weight |
| Titanium oxide | 8.0 % by weight |
| Kaoline | 26.5 % by weight |
| Boron nitride | 20.0 % by weight |

The blend composition was kneaded, and the kneaded composition was extrusion-molded in a fine line by means of an extruding machine. Further, the molded article t was heated by microwave (2.45 GHz, 700 W) for 3 minutes to obtain a light blue stick type cosmetic having a diameter of 2.0 mm.

### Example 9

### Blend composition:

| | |
|---|---|
| Tetraethoxysilane | 10.0 % by weight |
| 0.1 mol %-NaOH aqueous solution | 1.0 % by weight |
| Polyethylene glycol lauryl ether | 30.0 % by weight |
| Titanium dioxide-covered mica | 30.0 % by weight |
| Prussian blue | 10.0 % by weight |
| Kaoline | 19.0 % by weight |

The blend composition was kneaded, and the kneaded composition was extrusion-molded in a fine line by means of an extruding machine. The molded article was heated up to 80°C in the air and dried for 72 hours. Further, it was heated by microwave (2.45 GHz, 700 W) for 3 minutes to obtain a light blue pearl stick type cosmetic having a diameter of 2.0 mm.

### Example 10

### Blend composition:

| | |
|---|---|
| Titanium isopropoxide | 10.0 % by weight |
| 0.1 mol %-NaOH aqueous solution | 1.0 % by weight |
| Polyethylene glycol lauryl ether | 30.0 % by weight |
| Titanium dioxide-covered mica | 30.0 % by weight |
| Prussian blue | 10.0 % by weight |
| Kaoline | 19.0 % by weight |

The blend composition was kneaded, and the kneaded composition was extrusion-molded in a fine line by means of an extruding machine. The molded article was heated up to 50°C in the air and dried for 100 hours. Further, it was heated by microwave (2.45 GHz, 700 W) for 3 minutes to obtain a light blue pearl stick type cosmetic having a diameter of 2.0 mm.

### Comparative Example 1

### Blend composition:

| | |
|---|---|
| Tetraethoxysilane condensate | 50.0 % by weight |
| Boron nitride | 30.0 % by weight |
| Vinyl chloride | 20.0 % by weight |

The blend composition was kneaded by means of two rolls and further dispersed by means of three rolls, and then the kneaded composition was extrusion-molded in a fine line by means of a screw type extruding machine. The molded article was heated up to 700°C in nitrogen atmosphere and sintered for 5 hours to obtain a black sintered lead containing a carbon residue. Further, it was heated up to 700°C in the air and sintered for 5 hours, and it was further heated up to 1000°C in nitrogen atmosphere and sintered for 5 hours to obtain a white lead. Next, it was impregnated with a light blue ink to obtain a light blue stick type cosmetic having a diameter of 2.0 mm.

### Comparative Example 2

### Blend composition:

| | |
|---|---|
| Tetraethoxysilane condensate | 20.0 % by weight |
| 0.1 mol %-NaOH aqueous solution | 1.0 % by weight |
| Boron nitride | 39.0 % by weight |
| Ultramarine blue | 28.0 % by weight |
| Titanium oxide | 12.0 % by weight |

The blend composition was kneaded, and the kneaded composition was extrusion-molded in a fine line by means of an extruding machine and dried in the air at 80°C for 24 hours. Then, the molded article was heated up to 700°C in nitrogen and sintered for 5 hours to obtain a light blue pearl stick type cosmetic having a diameter of 2.0 mm.

### Comparative Example 3

### Blend composition:

| | |
|---|---|
| Titanium isopropoxide condensate | 20.0 % by weight |
| 0.1 mol %-NaOH aqueous solution | 1.0 % by weight |
| Boron nitride | 39.0 % by weight |
| Ultramarine blue | 28.0 % by weight |
| Titanium oxide | 12.0 % by weight |

The blend composition was kneaded, and the kneaded composition was extrusion-molded in a fine line by means of an extruding machine and dried in the air at 80°C for 24 hours. Then, the molded article was heated up to 700°C in nitrogen and sintered for 5 hours to obtain a light blue pearl stick type cosmetic having a diameter of 2.0 mm.

### Comparative Example 4

### Blend composition:

| | |
|---|---|
| Titanium dioxide-covered mica | 20.0 % by weight |
| Boron nitride | 40.0 % by weight |
| Kaoline | 9.0 % by weight |
| Microcrystalline wax | 1.0 % by weight |
| Liquid paraffin | 10.0 % by weight |
| Perfluoropolyether | 10.0 % by weight |
| Prussian blue | 10.0 % by weight |

The blend composition was mixed and dispersed, and the mixture was extrusion-molded in a fine line by means of an extruding machine and dried in the air at 50°C for 24 hours to obtain a light blue pearl stick type cosmetic having a diameter of 2.0 mm.

The respective stick type cosmetics (leads) obtained in Examples 1 to 10 and Comparative Examples 1 to 4 described above were evaluated for a mesopore diameter, a molding property, mechanical strength, a drawn line color (color developing property) and applying feeling by sensory evaluation.

The mechanical strength was shown by a ratio in which a fracture load (7N) of Comparative Example 1 was set at 100, wherein a three point flexural test was carried out for 20 leads of the respective leads under the conditions of 20 mm between supporting points and a speed of 5 mm/min, and it is shown that the higher the average value is, the better the mechanical strength is. The drawn line color and the applying feeling were evaluated by the form of a pencil built into the wood case.

The results thereof are shown in the following Table 1.

**Table 1**

| | Mesopore diameter | Molding property | Mechanical strength | Color developing property | Applying feeling |
|---|---|---|---|---|---|
| Example 1 | 50 nm | Good | 120 | Good color developing | Dry |
| Example 2 | 100 nm | Very good | 130 | Good color developing | Dry |
| Example 3 | 300 nm | Very good | 200 | Good color developing | Dry |
| Example 4 | 300 nm | Good | 160 | Good color developing | Dry |
| Example 5 | 400 nm | Good | 152 | Good color developing | Dry |
| Example 6 | 500 nm | Very good | 145 | Good color developing | Dry |
| Example 7 | 100 nm | Good | 120 | Good color developing | Dry |
| Example 8 | 100 nm | Good | 115 | Strong color developing | Smooth |
| Example 9 | 100 nm | Good | 110 | Good color developing | Smooth |
| Example 10 | 100 nm | Good | 105 | Good color developing | Smooth |
| Comparative Example 1 | -*1 | Good | 100 | Somber | Dry |
| Comparative Example 2 | -*1 | Bad (disintegration) | 80 | Good color developing | Liable to break |
| Comparative Example 3 | -*1 | Bad (disintegration) | 65 | Good color developing | Liable to break |
| Comparative Example 4 | -*1 | Normal | 30 | Normal | Slimy |

| | | | | | |
|---|---|---|---|---|---|
| *1: scarcely present or scarcely observed (= incapable of measuring) | | | | | |

As apparent from the results shown in Table 1 described above, it has been found that the stick type cosmetics prepared in 1 to 8 are liable to be abraded and provides dry applying feeling and that they have a very good color developing property and are excellent in mechanical strength. Also, it has become clear that in Examples 9 and 10 in which titanium dioxide-covered mica was blended, pearl stick type cosmetics having good color development are obtained without loosing a pearl color and that they are excellent in a molding property.

Further, to compare and weigh the stick type cosmetics prepared in Examples 3 to 6, it has been found that strength, an abrasion amount and applying feeling can freely be set by changing a blend amount and a molecular weight of the organic template and that characteristics corresponding to the aimed stick type cosmetics can readily be exhibited.

In contrast with this, the inorganic polymer was used as the binder and the molding auxiliary agent in Comparative Examples 1 to 3 falling outside the scope of the present invention, and therefore when the inorganic polymer was polycondensed to enhance the viscosity and the blend amount was increased in order to improve the molding property, the abrasion amount and the line intensity were decreased. Also, when the inorganic polymer was reduced in a viscosity or decreased in a blend amount in order to enhance the abrasion amount and the line intensity, the molding property was deteriorated.

Further, when wax was used, as shown in Comparative Example 4, in place of the inorganic polymer, slimy applying feeling of the wax appeared, and as a result, the dry applying feeling was lost.

### Industrial Applicability

In the present invention, obtained are a stick type cosmetic which maintains mechanical strength while providing soft and good applying feeling and which has a vivid color developing property and line intensity and can actualize multicolor and a production process for the same. Accordingly, it can suitably used, for example, for eye shadows and eyeliners.

## Claims

1. A stick type cosmetic comprising a mesoporous material as a skeleton.

2. The stick type cosmetic as described in claim 1, wherein the mesoporous material is at least one selected from the group consisting of oxide ceramics, nitride ceramics, phosphate ceramics, carbide ceramics, silicate ceramics and boride ceramics.

3. The stick type cosmetic as described in claim 1, wherein the mesoporous material is a composite material of at least one selected from the group consisting of oxide ceramics, nitride ceramics, phosphate ceramics, carbide ceramics, silicate ceramics and boride ceramics and an organic substance and/or a metal.

4. The stick type cosmetic as described in any one of claims 1 to 3, wherein a mesopore of the mesoporous material has a diameter falling in a range of 2 nm to 1000 nm.

5. The stick type cosmetic as described in any one of claims 1 to 4, wherein it is s cosmetic pencil.

6. A production process for a stick type cosmetic, wherein the stick type cosmetic as described in any one of claims 1 to 5 is produced by synthesizing a mesoporous material at low temperature by means of microwave heating or microwave heating and ultrasonic cleaning.
